# EUROPEAN PATENT APPLICATION

(11) **EP 1 431 270 A1**
(43) Date of publication of application: **23.06.2004**
(21) Application number: 03017696.0
(22) Date of filing: 18.08.2003
(51) Int. Cl.: C07C 201/12, C07C 201/16, C07C 205/45

(54) **A process for preparing 2-Nitro-4'-Fluorobenzophenone**

(30) Priority: 20.12.2002 US 324914
(71) Applicant: EASTMAN CHEMICAL COMPANY, Kingsport, TN 37660 (US)
(72) Inventor: Maleski, Robert Joseph, Kingsport, TN 37663 (US)
(74) Representative: Wibbelmann, Jobst, Dr., Dipl.-Chem.

(57) **Abstract**

The present invention is an improved process for preparing 2-nitro-4'-fluorobenzophenone which comprises contacting 2-nitrobenzoyl chloride and fluorobenzene in a reaction medium the presence of anhydrous ferric chloride. The process is typically performed at a temperature of -20°C to 25°C.

## Description

### Field of the Invention

Disclosed is an improved process for making 2-nitro-4'-fluorobenzophenone in which 2-nitrobenzoyl chloride is contacted with fluorobenzene in the presence of anhydrous ferric chloride.

### Background of the Invention

2-Amino-4'-fluorobenzophenone is an important intermediate used to prepare several pharmaceutical substances. Disclosed methods for its preparation, however, are not economically appealing. It has been made by several pathways, including by a Hoffman degradation of a carboxamide precursor by Suzuki et al, J. Org. Chem., 26, 2239, 1961. In addition, it has been synthesized by Hoesch Reaction of aniline with 4-halobenzonitriles. See JP 63275548 A2. Further, US Patent 5,053,543 describes a reaction of halobenzenes with N-alkoxy-N-alkyl anthranilic acid amides in the presence of an alkyl lithium reagent. WO 00/05195 also describes an aluminum chloride promoted reaction of halobenzenes and the p-toluenesulfonamide of anthranilic acid.

All of the forgoing methods suffer from expensive starting materials and/or complicated and expensive chemical transformations. Thus, Hino et al., Chem. Pharm. Bull. 1989, 110, describe an aluminum chloride promoted reaction between 2-nitrobenzoyl chloride and halobenzenes followed by reduction of the nitro group. See Scheme 1.

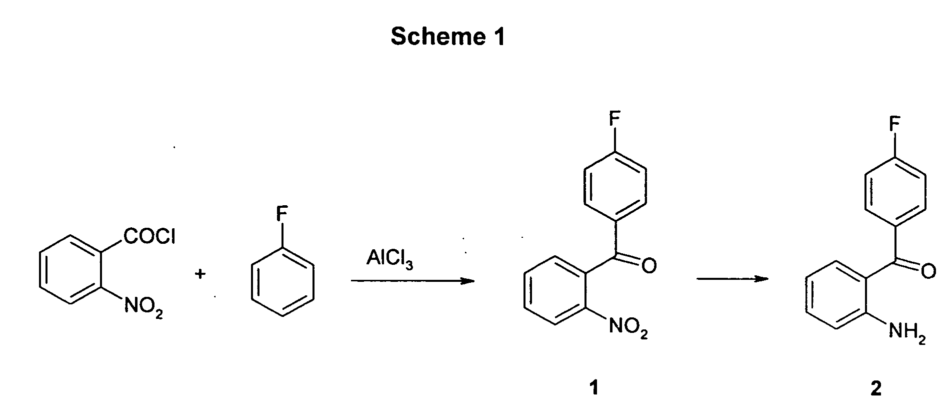

The procedure of Hino for the nitro-containing precursor compound 1 is not useful for large-scale synthesis of the desired end-product, compound 2. In particular, the process presents difficulties in solvent recovery and a need for column chromatography to recover compound **1**. Further, repetition of the Hino procedure failed to provide the intermediate. nitro compound at the claimed yield, and the resulting product was contaminated with large amounts of tarry, unidentified impurities, making purification of the desired product very difficult. (See Comparative Example 1.)

Thus, an efficient process that avoids chromatography and produces a purer product in higher yield would greatly reduce the cost of both the intermediate nitro compound **1** and the final desired amine **2**.

### Brief Summary of the Invention

The present invention is an improved process for preparing 2-nitro-4'-fluorobenzophenone which comprises contacting 2-nitrobenzoyl chloride and fluorobenzene in a reaction medium in the presence of anhydrous ferric chloride. The process is typically performed at a temperature of -20°C to 25°C.

### Detailed Description

The present invention offers significant improvement over current routes to 2-nitro-4'-fluorobenzophenone, offering both improved yield and purity of the title compound over other methods for its production. In addition, the present invention results in a simplified process for isolating 2-nitro-4'-fluorobenzophenone. As stated above, the presently claimed invention is an improved process for preparing 2-nitro-4'-fluorobenzophenone which comprises contacting 2-nitrobenzoyl chloride and fluorobenzene in a reaction medium in the presence of anhydrous ferric chloride. The process is performed under conditions and at a temperature conducive to formation of the title product.

The 2-nitrobenzoyl chloride and fluorobenzene starting materials are commercially available or may easily be made by processes known to those of skill in the art. Those starting materials are subjected to a condensation step in which they are contacted in a reaction medium using anhydrous ferric chloride as the condensation agent to produce a crude 2-nitro-4'-fluorobenzophenone product. The resulting crude product may be isolated by extraction, followed by crystallization. Good yields of high quality product are obtained.

Any solvent commonly used in Friedel-Crafts condensations can be used for the reaction, and may be used in any convenient amount that will allow the condensation reaction to proceed but not present undue difficulties in later extraction and recovery steps. Such solvents include carbon disulfide, nitrobenzene, dichloromethane, 1,1-dichloroethane, or 1,2-dichloroethane. Fluorobenzene can also be used in excess, in which case it would serve as the solvent for the reaction. Preferred solvents for the present invention are 1,2-dichloroethane or fluorobenzene.

When fluorobenzene is used as the solvent, it may be used at 2 to 6 times molar excess (relative to the 2-nitrobenzoyl chloride), with 2 to 3 times molar excess preferred. When a solvent other than fluorobenzene is used, lower levels of fluorobenzene are advantageous; for example, a molar excess of fluorobenzene, as compared to the nitrobenzoyl starting material, of from 0.75 to 2 molar excess, with 0.9 to 1.25 molar excess preferred. The amount of 2-nitrobenzoyl chloride relative to the reaction solvent may be any convenient amount, but typically should be 0.2 to 0.7 parts per part of solvent with 0.3 to 0.6 preferred.

For good performance the anhydrous ferric chloride should be present in molar amounts of 0.75 to 2.0, relative to the 2-nitrobenzoyl chloride; preferred amounts of ferric chloride are 0.9 to 1.1 molar as compared to the 2-nitrobenzoyl starting material. The anhydrous ferric chloride is typically added to a reaction medium comprising fluorobenzene, solvent and 2-nitrobenzoyl chloride, but alternative modes of addition, such as adding the 2-nitrobenzoyl chloride to the solvent, fluorobenzene, and ferric chloride, are also possible. The condensation step is best carried out at low temperature such as -20 to +25°C, with temperatures of -5 to +10°C being preferred. Any convenient pressure is appropriate, with atmospheric pressure being preferred. The reaction time depends on the concentration of reactants and on the temperature employed for the reaction. Typical reaction times are 0.25 to 2 hr, with 0.5-1.5 hr preferred.

As stated above, the resulting crude product may be isolated by extraction with an appropriate extraction solvent, followed by crystallization from that extraction solvent. Thus, when the reaction is complete, the reaction solvent is recovered for re-use (e.g., recycled), and the title product purified by crystallization. The foregoing is accomplished by first adding water to dissolve any iron salts present in the reaction medium. The amount of water used can be any convenient amount sufficient to dissolve the iron salts and carry out the solvent as its water azeotrope. Typically, 2 to 10 parts of water per part of product are used, with 4 to 8 parts preferred.

The resulting mixture is then distilled to remove an azeotropic composition comprising water and the reaction solvent. The temperature of the distillation step is determined by the boiling point of the azeotropic mixture of the solvent and water. For instance, when 1,2-dichloroethane is used as solvent, the temperature of the reaction medium at the beginning of the distillation is about 72° C and proceeds to about 100°C, the boiling point of water. The solvent in the water-containing distillate, i.e., the azeotrope, is recycled for recovery by separating it from the water, and after appropriate additional treatment it can be re-used in subsequent reactions.

Subsequently, a water-immiscible extraction solvent is added to the remaining reaction medium, which will still contain an amount of water after removal of the azeotrope. Addition of the extraction solvent provides a two-layer mixture (i.e., an organic layer and an aqueous or water-containing layer). The water layer, which contains iron salts, is removed and discarded. The title product remains in solution with the organic extraction solvent, which may be subjected to additional washings with water to remove any remaining salts. Thereafter, the product-containing extraction solvent is subjected to a crystallization step by cooling. Typically, the solution should be cooled to about 0° to about 25° C to bring about precipitation of the product. The resulting solid may then be filtered and further washed to remove remaining impurities with an appropriate solvent. In addition, the filtered and washed resulting solid may then be dried.

The solvent used to extract the product should be one that is immiscible with water and easily dissolves the product, and which would allow the product-containing solution to be washed with water to free it of undesired salts. Further, the extraction solvent should be one from which the product precipitates in acceptable purity upon cooling. Typical solvents include C4-C8 alcohols such as n-octanol, n-hexanol, n-pentanol, sec butyl alcohol, isobutyl alcohol, and n-butyl alcohol, with isobutyl alcohol and n-butyl alcohol being preferred. The amount of such solvents used to perform the extraction may be any convenient amount that will allow for good phase separation, along with acceptable yield and purity of the final product. Usual amounts of such solvents are 1 to 4 parts solvent per part of product, with 1.5 to 2.5 parts solvent per part of product being preferred.

Likewise, the solvent used to wash the crystallized product can be any solvent that will dissolve reaction impurities, but not the product. Typical of such solvents are isopropyl alcohol, ethyl alcohol, and methyl alcohol, with methyl alcohol preferred. The solvent used for the extraction step and from which the product is crystallized may also be used to wash the product, provided the washing is performed at a suitable temperature (e.g., so as not to dissolve the product).

This invention can be further illustrated by the following examples, although it will be understood that these examples are included merely for purposes of illustration and are not intended to limit the scope of the invention unless otherwise specifically indicated.

### EXAMPLES

### Comparative Example 1

(Procedure of Hino, et al.) A solution of 18.6 g of 2-nitrobenzoyl chloride (0.1 moles) in 100 mL of fluorobenzene was cooled to 0-5°C and treated with 18 g (0.134 moles) of aluminum chloride added over a period of 1 hr. The reaction was held at 0-5°C for 1 hour and was downed onto ice, the product was extracted into ethyl acetate, and the layers were separated. The ethyl acetate layer was evaporated to generate 23 g of a semi-solid. Much tar was present in the residue. The semi-solid was recrystallized from methyl alcohol to give 7.8 g (32%) of a dark solid that assayed 95% by NMR.

### Example 1

A solution of 24 g of 2-nitrobenzoyl chloride (0.129 moles) and 19 g (0.2 moles) of fluorobenzene in 50 mL of 1,2-dichloroethane was cooled to 0-5°C and treated with 24 g (0.147 moles) of anhydrous ferric chloride added over a period of 0.5 hr. After stirring for 1 hr the reaction mass was added to 250 mL of ice and water, and the 1,2-dichloroethane was removed as its water azeotrope up to a pot temperature of 90°C. The reaction mass was cooled to 75°C and 75 mL of isobutyl alcohol was added. The agitation was stopped, and the lower layer, containing most of the iron salts, was removed through a bottom stopcock. The upper layer was washed twice at 75-80°C with 200 mL of water, and the upper layer was cooled to room temperature. The product crystallized during this cooling. After stirring the product slurry for 1 hr, it was filtered, and the product was washed with 100 mL of methanol and dried to give 23.9g (75.5%) of light tan product, which assayed >98% by nmr.

### Comparative Example 2

A solution of 18.6 g of 2-nitrobenzoyl chloride (0.1 moles) in 50 mL of fluorobenzene (0.52 moles) was cooled to 0-5°C and treated with 15 g (0.112 moles) of aluminum chloride added over a period of 1 hr. After stirring for 1 hr the reaction mass was added to 100 mL of water, and the fluorobenzene was removed by distillation as it water azeotrope up to a pot temperature of 99°C. The reaction mass was cooled to 75°C and extracted with 200 mL of ethyl acetate. The ethyl acetate solution was separated from the water solution, and washed twice with 200 mL of water. After evaporation of the ethyl acetate, about 26 g of a semi-solid was obtained. The residue was recrystallized from 100 mL of iso-butanol to give 9.1 g (37%) of a dark black solid, assay 94% by NMR.

### Example 2

The procedure of Example 1 was repeated except that n-butanol was used in the place of iso-butanol for the extraction and crystallization. The product was obtained in 70% yield (22g) and 96% assay (by nmr).

### Example 3

A solution of 74.9g of 2-nitrobenzoyl chloride (0.4 moles) and 50 g (0. 52 moles) of fluorobenzene in 100 mL of 1,2-dichloroethane was cooled to 0-5°C and treated with 72 g (0.45 moles) of anhydrous ferric chloride, which was added in 3 equal portions over a period of 1 hr. After stirring for 1 hr the reaction mass was added to 400 mL of water, allowing the temperature to rise. The pot temperature was increased to 95°C, during which time the 1,2-dichloroethane was removed as its water azeotrope. The reaction mass was cooled to 75°C and 300 mL of isobutyl alcohol was added. The agitation was stopped, and the lower layer, containing most of the iron salts, was removed through a bottom stopcock. The upper layer was washed twice at 75-80°C with 300 mL of water, and the upper layer was cooled to room temperature. The product crystallized during this cooling. After stirring the product slurry for 1 hr, it was filtered, and the product was washed with 200 mL of methanol and dried to give 77.4g (78.8%) of light tan product, which assayed >100% by HPLC.

### Example 4

The procedure of Example 1 was repeated but only 11.5 g (.071 moles) of anhydrous ferric chloride was used. High assay product (100% by HPLC) was obtained in 53% yield (16.8g).

### Example 5

A solution of 37.1 g of 2-nitrobenzoyl chloride (0.20 moles) and 102g (1.06 moles) of fluorobenzene was cooled to 0-5°C and treated with 36 g (0.22 moles) of anhydrous ferric chloride added over a period of 0.5 hr. After stirring for 1 hr the reaction mass was added to 200 mL of ice and water, and the fluorobenzene was removed as its water azeotrope up to a pot temperature of 90C. The reaction mass was cooed to 75°C and 100 mL of isobutyl alcohol was added. The agitation was stopped, and the lower layer, containing most of the iron salts, was removed through a bottom stopcock. The upper layer was washed twice at 75-80 with 200 mL of water, and the upper layer was cooled to room temperature. The product crystallized during this cooling. After stirring the product slurry for 1 hr, it was filtered, and the product was washed with 100 mL of methanol and dried to give 31.3g (64%) of light tan product, which assayed >99.8% by HPLC.

## Claims

1. A process for preparing 2-nitro-4'-fluorobenzophenone which comprises contacting 2-nitrobenzoyl chloride and fluorobenzene in a reaction medium the presence of anhydrous ferric chloride at a temperature of -20°C to 25°C to form 2-nitro-4'-fluorobenzophenone.

2. A process according to Claim 1, wherein the reaction medium further comprises a solvent selected from the group consisting of fluorobenzene used in molar excess, carbon disulfide, nitrobenzene, dichloromethane, 1,1-dichloroethane, and 1,2-dichloroethane.

3. A process according to Claim 2 wherein the solvent is 1,2-dichloroethane or fluorobezene or a mixture thereof.

4. A process according to Claim 3 wherein the temperature is from -5°C to 10°C.

5. A process according to Claim 2 wherein the process further comprises the steps of adding water to the reaction medium and distilling the reaction medium to remove a distillate comprising water and the solvent.

6. A process according to Claim 5 wherein the process further comprises the steps of adding a water-immiscible extraction solvent to the reaction medium to form a two-phase mixture comprising an organic layer, which comprises extraction solvent and 2-nitro-4'-fluorobenzophenone, and an aqueous layer, discarding the aqueous layer, and cooling the organic layer to a temperature of 0° to 25°C to crystallize the 2-nitro-4'-fluorobenzophenone.

7. A process according to Claim 6 wherein the extraction solvent is selected from the group consisting of n-octanol, n-hexanol, n-pentanol, sec butyl alcohol, isobutyl alcohol, and n-butyl alcohol.

8. A process according to Claim 6 wherein the extraction solvent is isobutyl alcohol or n-butyl alcohol.

9. A process according to Claim 6, wherein the process further comprises filtering the 2-nitro-4'-fluorobenzophenone from the extraction solvent and drying the 2-nitro-4'-fluorobenzophenone.

10. A process for preparing 2-nitro-4'-fluorobenzophenone which comprises the steps of:
contacting 2-nitrobenzoyl chloride and fluorobenzene in a reaction medium the presence of anhydrous ferric chloride and a solvent selected from the group consisting of carbon disulfide, nitrobenzene, dichloromethane, 1,1-dichloroethane and 1,2-dichloroethane at a temperature of -5°C to 10°C to form a product comprising 2-nitro-4'-fluorobenzophenone;
adding water to the reaction medium;
distilling the reaction medium to remove a distillate comprising water and solvent;
adding a water-immiscible extraction solvent to the reaction medium to form a two-phase mixture comprising an organic layer, comprising extraction solvent and 2-nitro-4'-fluorobenzophenone, and an aqueous layer;
discarding the aqueous layer; and
cooling the organic layer to a temperature of 0° to 25°C to crystallize 2-nitro-4'-fluorobenzophenone.

11. A process according to Claim 10 wherein the process further comprises the steps of filtering and drying the 2-nitro-4'-fluorobenzophenone product, and wherein the solvent is 1,2-dichloroethane and the extraction solvent is isobutyl alcohol or n-butyl alcohol.
